# EUROPEAN PATENT APPLICATION

(11) **EP 4 257 616 A1**
(43) Date of publication of application: **11.10.2023**
(21) Application number: 21899523.1
(22) Date of filing: 19.05.2021
(51) Int. Cl.: C07K 19/00, C07K 16/46, A61K 39/395, A61P 35/00

(54) **TRIFUNCTIONAL FUSION PROTEIN CONTAINING TUMOR-ASSOCIATED ANTIGEN (TAA) ANTIBODY AND APPLICATION THEREOF**

(30) Priority: 04.12.2020 CN 202011406572
(71) Applicant: Qure Biotechnology (Shanghai) Co., Ltd., Shanghai 201220 (CN)
(72) Inventor: QU, Xiangdong, Shanghai 200120 (CN)
(74) Representative: Cabinet Laurent & Charras
(86) International application number: PCT/CN2021/094631
(87) International publication number: WO 2022/116480

(57) **Abstract**

Provided are a trifunctional fusion protein containing a tumor-associated antigen (TAA) antibody, a TGF-β inhibitor, and a CD3 antibody, and an application thereof. By introducing a TGF-β inhibitor into antibody molecule, the trifunctional fusion protein can specifically target the antibody molecule to tumors by means of a TAA antibody having high affinity; an anti-CD3 antibody can play a T cell killing role; and the TGF-β inhibitor can block a TGF-β signal, thereby promoting the infiltration and activation of T cells in a tumor microenvironment.

## Description

### TECHNICAL FIELD

The present invention belongs to the field of biomedicine, and relates to a trifunctional fusion protein containing tumor-associated antigen (TAA) antibody, TGF-β inhibitor, and CD3 antibody, and the application thereof.

### BACKGROUND

### 1. Tumor microenvironment

The tumor microenvironment (TME) is the environment surrounding the tumor, including blood vessels, immune cells, fibroblasts, signal molecules, and extracellular matrix (ECM). Tumor and the surrounding microenvironment are closely related and constantly interact with each other. Tumor can affect the microenvironment by releasing extracellular signals, promoting tumor angiogenesis and inducing immune tolerance, while the immune cells in the microenvironment can also affect the growth and differentiation of tumor cells.

### (1) Blood vessels

According to statistics, 80-90% of cancers are adenocarcinomas, or cancers derived from epithelial tissues. Such tumors do not undergo vascularization, resulting in tumor growth to greater than 2 mm in diameter only after neovascularization. Angiogenesis is up-regulated in the tumor microenvironment and provides nutrition to tumor cells. The vasculature formed by angiogenesis is different from that of normal tissues in many ways, mainly reflected in the enhanced permeability and retention effect (EPR) and hypoxic environment.

### (2) Immune cells

Myeloid-derived suppressor cell (MDSC) is the general term of a type of heterogeneous cell populations with the potential to suppress T cell responses. They can rapidly proliferate in tumors by regulating wound repair and inflammation, which is a phenomenon associated with the inflammatory response observable at most tumor sites. Tumor-associated macrophage (TAM) is one of the research hotspots in MDSC. TAMs are able to be recruited into tumors in response to tumor-associated inflammatory responses, but unlike normal macrophages, TAMs have no cytotoxic effects. Therefore, TAMs are important evidence of a strong link between chronic inflammation and cancer. In *in vitro* experiments, TAMs can be induced by administering different immune regulatory cytokines such as interleukin 4 (IL-4) and interleukin 13 (IL-13) to macrophage progenitor cells. TAMs gather in the necrotic area of the tumor, and make the tumor cells escape the monitoring of normal immune cells by secreting IL-10, and promote angiogenesis by secreting vascular endothelial growth factor (VEGF) and nitric oxide synthase (NOS), and promote tumor growth by secreting epidermal growth factor (EGF) to reconstruct ECM. The NF-κB signaling pathway of TAMs is inhibited, resulting in the exhibition of chronic inflammatory responses in the tumor microenvironment. An increase in TAMs is associated with a poor prognosis. TAMs represent potential targets for novel cancer therapies.

Neutrophils are polymorphonuclear immune cells that are an important part of the innate immune system. Neutrophils are enriched in tumors and certain cancers (e.g., lung adenocarcinoma) and are thought to be associated with worse prognosis. In some patients with solid tumors, the number of neutrophils (and myeloid cell precursors) in the blood may be increased. Experiments in mice have shown that tumor-associated neutrophils promote tumor growth, but there are also studies showing the opposite conclusion.

Tumor infiltrating lymphocytes (TILs) are lymphocytes that penetrate the tumor. TILs share a common origin with hematopoietic stem cells, but differentiate during development. Tumor cells can induce apoptosis of activated T lymphocytes by secreting exosomes containing death ligands FasL and TRAIL, etc., and turn off the normal cytotoxic response of NK cells by the same method.

### (3) Fibroblasts

Carcinoma associated - fibroblast (CAFs) are a population of fibroblasts of diverse origin whose function is "pirated" by tumor cells and leads to tumorigenesis. CAFs are usually derived from normal fibroblasts of the surrounding matrix, but can also be derived from pericytes, smooth muscle cells, fibrocytes, mesenchymal stem cells, or transformed by EMT and EndMT. In *in vitro* experiments, CAFs do not inhibit the growth of tumor cells, but can induce angiogenesis by secreting VEGFs, fibroblast growth factors (FGFs), platelet-derived growth factors (PDGFs) and other angiogenesis-promoting signals, thereby promoting tumor growth. CAFs can also secrete TGF-β to promote EMT, and tumor cells can transfer through EMT and participate in the inhibition of cytotoxic T cells and NKT cells. As fibroblasts, CAFs are also able to remodel the ECM to include more paracrine survival signals, such as IGF-1 and IGF-2, thereby promoting the survival of surrounding tumor cells. Studies have shown that CAFs are associated with reverse Warburg effects, and CAFs can perform aerobic glycolysis and feed lactic acid to tumor cells.

### 2. TGF-beta

Transforming growth factor beta (TGF-β) is a multifunctional cytokine belonging to the transform growth factor superfamily. TGF-β is present only in vertebrates, including three subtypes: TGF-β1, TGF-β2, TGF-β3, and other signal proteins. TGF-β is an essential component for the normal development of multiple organs and tissues such as the heart, eyes, and palate of vertebrates. It also has the functions of regulating the adaptive immune system and coordinating wound healing necessary for the long-term survival of humans and other higher vertebrates.

The three subtypes of TGF-β have high similarity in the primary structure, which homology reaches 70-80%. TGF-β1 has 390 amino acids, TGF-β2 and TGF-β3 each contain 412 amino acids. They all have a signal peptide necessary for secretion from cells at the N-terminus, a LAP domain and a C- terminal region consisting of 112-114 amino acid residues. This peptide segment is released from the original region by proteolysis and becomes a mature TGF-β molecule. The mature TGF-β protein dimerizes to generate a 25 KDa active protein. TGF-βs all have 9 cysteine residues, 8 of which form disulfide bonds within the molecule, thereby forming the structural feature of cysteine knot in the TGF-β superfamily. The ninth cysteine forms a disulfide bond with the corresponding cysteine of another TGF-β protein, thereby forming a functional dimer.

All leukocytes can produce the inactive form of TGF-β protein, where TGF-β is secreted as a complex with LTBP (latent TGF-beta binding protein) and LAP (latency-associated peptide), and serum proteases such as plasmin can catalyze the release of active TGF-β from the complex. This physiological phenomenon of active TGF-β release often occurs on the surface of macrophages: inflammatory stimulus signals activate macrophages and promote the activation of plasmin, resulting in the binding of the inactive TGF-β complex to CD36 through its ligand, thrombospondin-1 (TSP-1), which ultimately enhances the release of active TGF-β. Macrophages can also release active TGF-β into the extracellular fluid in a manner of engulfing IgG-bound inactive TGF-β complexes secreted by plasma cells.

TGF-β is activated in a cell- or tissue-specific manner, including proteases, integrin, pH, and reactive oxygen species. Disturbance of TGF-β activator leads to dysregulation of TGF-β signaling, resulting in a variety of complications, including inflammation, autoimmune diseases, tissue fibrosis, tumors, and cataract.

Signaling pathway and biological function:
Activated TGF-β is released and then forms a serine/threonine kinase complex with other factors, and finally binds to the TGF-β receptor to activate the downstream signaling pathway. The TGF-β receptor is composed of a type 1 receptor subunit and a type 2 receptor subunit. When TGF-β binds to the receptor, type 2 receptor kinase is phosphorylated and activate type 1 receptor kinase, thereby activating a signaling cascade. TGF-β receptor downstream signaling pathways can be divided into classical pathway (SMAD pathway) and DAXX dependent apoptosis pathway (DAXX pathway).
(1) Classical pathway: The activated TGF-β complex binds to the type 2 receptor subunit of the TGF-β receptor and then recruits and phosphorylates the type 1 receptor, which recruits and phosphorylates the receptor-regulated SMAD protein (R-SMAD). R-SMAD then combines with coSMAD protein (common SMAD, which is SMAD4 in mammals) to form a heterodimer and enters the nucleus, and plays a role in regulating gene expression as a transcription factor. The SMAD pathway is regulated by feedback inhibition, and SMAD6 and SMAD7 may block the type I receptor subunit. There are also a large number of studies having found that immune suppression function is dependent on TGF-β signaling through SMAD-3, so it is often considered to be related to tumorigenesis.
(2) TGF-β can induce apoptosis or programmed cell death in human lymphocytes and hepatocytes, while in TGF-β-deficient mice, autoimmune disorders may occur due to excessive proliferation of lymphocytes. Death-associated protein 6 (DAXX) can bind to death receptor Fas to cause apoptosis, and DAXX can also bind to the C-terminus of type 2 TGF-β receptor. DAXX is subsequently phosphorylated by homeodomain-interacting protein kinase 2 (HIPK2) to activate apoptosis signal-inducing kinase 1(ASK1), and ASK1 subsequently activates the Jun amino-terminal kinase (JNK) pathway, which ultimately leads to apoptosis.

The classical signaling pathway of TGF-β can activate different downstream substrates and regulatory proteins, and induce the proliferation, activation, differentiation and chemotaxis of immune cells, so it has been a research hotspot in the field of cancer, autoimmune diseases and infectious diseases.

### (1) Effects on T lymphocytes

TGF-β plays a crucial role in T cell regulation and differentiation. TGF-β1 can induce CD4+T cells to differentiate into inducible Tregs (iTregs) and Th17 cells that secrete pro-inflammatory cytokines. TGF-β1 can promote the up-regulation of Foxp3 expression and differentiation into Tregs of activated Th cells. In mouse models, this induction effect of TGF-β1 is confirmed to be age-related. At the same time, some studies have shown that neutralizing TGF-β1 can inhibit the differentiation of Th cells into Th17 cells *in vitro.* In other words, TGF-β1 plays an opposite role in the generation of Th17 cells to its traditional concept of being an anti-inflammatory cytokine, but it also implies that TGF-β1 can maintain autoimmune homeostasis by regulating the balance between inflammatory cells and anti-inflammatory cells.

### (2) Effects on B lymphocytes

TGF-β can inhibit B cell proliferation, of which the exact mechanism is still unclear, but some data show that TGF-β can inhibit B cell proliferation by inducing transcription factor Id3 to up-regulate p21, and inhibiting other key regulatory genes, such as c-myc and ATM. CD40, a key surface molecule that activates the innate immune response, can undo the TGF-β-induced B cell growth inhibition described above by inducing Smad7 expression. TGF-β also can block B cell activation, promote conversion of B cells to IgA class in humans and mice, and inhibit antibody generation.

### (3) Effects on macrophages

It is now generally accepted that TGF-β stimulates monocytes in resting state and inhibits activated macrophages. For monocytes, TGF-β has a chemoattractant effect and a pro-inflammatory response. However, it has also been reported that TGF-β can down-regulate the production of pro-inflammatory cytokines in monocytes and macrophages by inhibiting NF-κB signaling pathway. This seemingly paradoxical role of TGF-β suggests that the effect of TGF-β is highly dependent on the environment in which the immune cells are located at the time.

The upregulation of TGF-β plays an immunosuppressive role, which is often associated with the development of many malignancies. TGF-β may also be involved in many autoimmune diseases through dysregulation of immunosuppressive function.

### (1) Tumor

In normal cells, TGF-β stops the cell cycle and proliferation in G1 phase through signal pathways to induce differentiation or promote apoptosis. However, in many tumor cells, the TGF-β signaling pathway is mutated, and TGF-β loses control of the cell. Tumor cells proliferate, accompanied by proliferation of surrounding stromal cells (fibroblasts), both of which increase their TGF-β production. These TGF-β act on the surrounding stromal cells, immune cells, endothelial cells and smooth muscle cells, inhibit the immune response, promote angiogenesis, and thus make the tumor more invasive. TGF-β can also convert effector (immune) T cells, which are able to attack tumor cells via an inflammatory response, into regulatory (suppressor) T cells, thereby inhibiting the inflammatory response. In normal tissues, different cells express adhesion molecules and secrete cytokines, respectively. Tissue integrity can be maintained through cell-to-cell feedback, but these feedback mechanisms are disrupted in tumor tissues. When TGF-β signal fails to control NF-κB activity in tumor cells, malignant tumor cells can still survive in the presence of activated immune cells.

### (2) Obesity and diabetes

The TGF-β/SMAD3 signaling pathway is important in regulating glucose and energy metabolic homeostasis, which may also play a role in diabetic nephropathy. In spontaneously obese mice, loss of TGF-β signaling induces adiposity through inflammatory signaling.

In the presence of IL-2, iTregs induced by TGF-β inhibit the development of experimental autoimmune encephalomyelitis (EAE), an animal model of multiple sclerosis (MS), via Foxp3 and IL-10 mediated responses. It suggests that TGF-β and iTreg may play a role in the regulation and treatment of MS. Clinically, a decrease in TGF-β level is indeed observed in patients with multiple sclerosis. The role of TGF-β in regulating Th17 cell apoptosis may explain its role in multiple sclerosis: after TGF-β levels are reduced, apoptosis of Th17 cells cannot be induced, and excessive secretion of TNF-α induces the de-myelination of oligodendrocytes and neurons through TNFR1. Thus, a decrease in TGF-β level during MS may also prevent remyelination of neurons.

### 3. CD3 target-related

T lymphocytes, abbreviated as T cells, are derived from lymphoid stem cells from bone marrow that differentiate and mature in the thymus, then they are distributed throughout the immune organs and tissues throughout the body through lymphatic and blood circulation to exert immune function. T cells are highly effective killer cells that quickly destroy virus-infected cells and cancer cells. The killing effect of T cells requires the formation of immune synapses, which is highly dependent on the TCR recognition of the complex formed by MHC molecules on the surface of antigen presenting cells and the presented antigen peptides. The activated immune synapse will release cytotoxins and cytokines for killing effect. In the process of immune synapse formation, limited by the distance between T cells and target cells, the bispecific antibody directing T cells needs to mimic the formation of immune synapse. One end of that bridges the TCR receptor on T cell by targeting CD3, and the other end of that bridges the target cell by targeting the surface antigen of target cell.

The rationale for bispecific antibodies to direct cytotoxic t-cells to cancer cells was first described in 1985. Bispecific antibody-mediated T-cell redirected killing of tumor cells is considered to be the most promising cancer therapeutic strategy. In 2009, Removab, the first bispecific antibody drug in the world, was approved by the European Medicines Agency (EMA) for malignant ascites caused by EpCAM-positive tumors where standard treatment is ineffective or infeasible. Catumaxomab (anti-CD3 and anti-EpCAM), as the first FDA-approved bispecific antibody, aims to be a "trifunction" bispecific antibody that can not only bridge tumor target cells and T cells, but also bind to Fc γ receptor positive effector cells, macrophages, etc. As the first T cell recruiter bispecific antibody approved by the FDA, catumaxomab has a milestone significance. In fact, catumaxomab has indeed shown good efficacy in the treatment of ovarian cancer and other cancer patients with ascites. However, during this process, side effects such as fever, nausea and vomiting caused by cytokine storm often occur. At the same time, after four injections of 10, 20, 50, 150 µg on days 0, 3, 7 and 10 respectively, 70% of patients developed Human anti-mouse antibody/human anti-rat antibody (HAMA/HARA). These side effects may be related to the following factors: cytokine storm caused by the high affinity of bispecific antibodies to CD3 with low activation of T cells before binding to tumor cells; cytokine storm caused by excessive activation of T cells after bispecific antibodies binding to tumor cells; potential non-specific killing of normal tissues by bispecific antibodies caused by considerable expression of EpCAM in some normal tissues.

Blinatumomab is the second T cell recruiter bispecific antibody approved by FDA. BiTE^{®} technology was used to design it. ScFVs of two monoclonal antibodies against CD19 and CD3 were connected with a "GGGGS" in this bispecific antibody, and it was used to treat indications (relapsing ph-ALL) through T cell mediated lysis, cytotoxicity by released cytokines. Due to the removal of Fc, this bispecific antibody has no ADCC and CDC function. Because of the small molecular weight and short half-life, only less than 1% of treated patients have an ADA response. However, due to T cell activation, a considerable number of patients still have side effects caused by cytokine storm. At the same time, since the half-life of Blinatumomab in serum is only 1.25+/-0.63 hours, infusion bump is required for intravenous administration, which has certain inconveniences in clinical use. The safety and effectiveness balance requirements of the Blinatumomab application requirements have been met by the selected technology platform and structural model, but the PK requirements of the druggable part have not been met. It is also the biggest limitation of the BiTE^{®} platform in clinical application.

In recent years, multinational pharmaceutical companies such as Roche, Johnson & Johnson, Amgen, Pfizer and Takeda have invested heavily to continuously increase the development of bispecific antibodies. Currently, the design of bispecific antibodies varies from 25KD nanobodies to ≥ 150KD IgG-like fusion proteins. The advantage of the small molecular weight structure is that it allows the bridged cells to be closer together and has better tumor tissue permeability. The IgG-like design favors the half-life of the antibody and the killing effect induced by Fc.

### DISCLOSURE

The prior art lacks a trifunctional fusion protein comprising a tumor-associated antigen (TAA) antibody, a TGF-β inhibitor, and a CD3 antibody. The purpose of the present invention is to provide a trifunctional fusion protein comprising a tumor-associated antigen (TAA) antibody, a TGF-β inhibitor, and a CD3 antibody, and applications thereof.

In order to achieve the above object, the present invention provides a trifunctional fusion protein comprising a tumor-associated antigen (TAA) antibody, a TGF-β inhibitor and a CD3 antibody.

Optionally, the fusion protein has the following general structure:
{[TAA antibody]n, [CD3 antibody]x, [TGF-β inhibitory molecule]m}, wherein, n>=1, x = 1, m>=1;
wherein,
the [TAA]n refers to an antibody or polypeptide fragment that binds to a tumor-associated antigen in a monovalent or multivalent manner;
the [CD3]x refers to an antibody or polypeptide fragment that binds to CD3 in a monovalent manner;
the [TGF-β inhibitory molecule]m refers to an antibody or polypeptide fragment that binds to TGF-β in a monovalent or multivalent manner.

Optionally, the target of the TAA antibody includes: CD20, CD19, CD38, CD30, CD52, slamf7, GD2, CD24, CD47, CD133, CD239, CD276, CEA, Epcam, Trop2, TAG72, MUC1, MUC16, mesothelin, folrl, CLDN18.2, PDL1, EGFR VIII, C-MET, HER2, FGFR2, FGFR3, PSMA, PSCA, EphA2, ADAM17, 17-A1, NKG2D ligands, MCSP, LGR5, SSEA3, SLC34A2, BCMA, or GPNMB;
the target of the CD3 antibody includes: OKT3, SP34 or UCTH1;
the TGF-β inhibitor includes: human TGF-βRII extracellular region and a derivative thereof or human TGF-βRI extracellular region and a derivative thereof.

Optionally, the fusion protein further comprises an FC segment; the FC segment is selected from the group consisting of Human IgG1 FC, Human IgG2 FC, Human IgG3 FC, Human IgG4 FC or a variant thereof; and the FC segment comprises an A chain and a B chain, and the variant includes the following forms:

| Combination number | FC | Heterodimer mutation (Eu numbering) |
|---|---|---|
| 1 | FC-A chain | T366Y |
| | FC-B chain | Y407T |
| 2 | FC-A chain | T366W |
| | FC-B chain | T366S/L368A/Y407V |
| 3 | FC-A chain | S354C/T366W |
| | FC-B chain | Y349C/T366S/L368A/Y407V |
| 4 | FC-A chain | S364H/F405A |
| | FC-B chain | Y349T/T394F |
| 5 | FC-A chain | T350V/L351Y/F405A/Y407V |
| | FC-B chain | T350V/T366L/K392L/T394W |
| 6 | FC-A chain | K392D/K409D |
| | FC-B chain | E356K/D399K |
| 7 | FC-A chain | ID221E/P228E/L368E |
| | FC-B chain | D221R/P228R/K409R |
| 8 | FC-A chain | K360E/K409W |
| | FC-B chain | Q347R/D399V/F405T |
| 9 | FC-A chain | K360E/K409W/Y349C |
| | FC-B chain | Q347R/D399V/F405T/S354C |
| 10 | FC-A chain | K370E/K409W |
| | FC-B chain | E357N/D399V/F405T |
| 11 | FC-A chain | F405L |
| | FC-B chain | K409R |
| 12 | FC-A chain | K360D/D399M/Y407A |
| | FC-B chain | E345R/Q347R/T366V/K409V |
| 13 | FC-A chain | Y349S/K370Y/T366M/K409V |
| | FC-B chain | E356G/E357D/S364Q/Y407A |
| 14 | FC-A chain | L351D/L368E |
| | FC-B chain | L351K/T366K |
| 15 | FC-A chain | GQPFRPEVHLLPPSREEMTKNQVSLTCLARGFYPKDIAV EWESNGQPENNYKTTPSRQEPSQGTTTFAVTSKLTVDK SRWQQGNVFSCSVMHEALHNHYTQKTISL |
| | FC-B chain | GQPREPQVYTLPPPSEELALNELVTLTCLVKGFYPSDIA VEWLQGSQELPREKYLTWAPVLDSDGSFFLYSILRVAA EDWKKGDTFSCSVMHEALHNHYTQKSLDR |
| 16 | FC-A chain | L368D/K370S |
| | FC-B chain | E357Q/S364K |
| 17 | FC-A chain | S354C/T366W/K409A |
| | FC-B chain | Y349C/T366S/L368A/Y407V/F405K |
| 18 | FC-A chain | S354C/T366W/F405K/K360EQ347E |
| | FC-B chain | Y349C/T366S/L368A/Y407V/Q347R/T394W |
| 19 | FC-A chain | T366W/K409A |
| | FC-B chain | T366S/L368G/Y407A/F405K |
| 20 | FC-A chain | knobs(T366W/F405K) |
| | FC-B chain | holes(T366S/L368G/Y407A/K409A) |
| 21 | FC-A chain | Q347A/S364K/T366V/K370T/K392Y/F405S/Y407V/K409W/ T411N |
| | FC-B chain | Q347E/Y349A/L351F/S364T/T366V//K370T/T394D/V397L/ D399E/D401Q/F405A/Y407S/K409R/T411R |
| 22 | FC-A chain | K274Q/N276K/Y300F/A339T/Q347A/S364K/T366V/K370T/ N384S/K392Y/V397M/F405S/Y407V/K409W/T411N/V422I/ H435R/Y436F |
| | FC-B chain | Q347E/Y349A/L351F/S364T/T366V//K370T/T394D/V397L/ D399E/D401Q/F405A/Y407S/K409R/T411R |

Optionally, the trifunctional fusion protein comprises: an FC segment, a TAA antibody, two TGF-β inhibitors, and a CD3 antibody; the TAA antibody comprises: a first TAA antibody and a second TAA antibody, wherein the first TAA antibody comprises a Fab domain, and the second TAA antibody and the CD3 antibody are scFvs; the FC segment comprises an A chain and a B chain; wherein the N-terminus of the A chain is linked to the first TAA antibody, and the C-terminus of that is linked to one TGF-β inhibitor; the N-terminus of the B chain is linked to the second TAA antibody, and the C-terminus of that is linked to the other TGF-β inhibitor; and the CD3 antibody is linked to the second TAA antibody.

Optionally, the target of the TAA antibody is CLDN18.2, the target of the CD3 antibody is OKT3; and the TGF-β inhibitor is the human TGF-βRII extracellular region.

Optionally, the fusion protein is obtained by the fusion of SEQ ID NOs: 13, 14, and 15.

Optionally, the fusion protein further comprises: a linker sequence, which comprises one or more G4S.

The present invention also provides a use of the above-mentioned trifunctional fusion protein comprising a tumor-associated antigen (TAA) antibody, a TGF-β inhibitor and a CD3 antibody in the preparation of a medicament for inhibiting or treating cancer.

The cancer includes: myeloma, lymphoma, leukemia, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, or myelodysplastic syndrome, or a cancer or tumor derived from: colorectal, breast, ovary, pancreas, stomach, prostate, kidney, cervix, thyroid, endometrium, uterus, bladder, neuroendocrine, head and neck, liver, nasopharynx or testis.

Compared with the prior art, the present invention has the following beneficial effects:
(1) The trifunctional fusion protein of the present invention introduces a TGF-β inhibitor into the antibody molecule, in addition to being able to specifically target the antibody molecule to tumors through high-affinity TAA, and the anti-CD3 antibody can play a role in killing T cells, and at the same time TGF-β inhibitors can block TGF-β signaling and promote the infiltration and activation of T cells in the tumor microenvironment.
(2) In the present invention, the effective dose of the anti-CD3 antibody can be adjusted to be equivalent to that of TGF-βRII by reducing the affinity of the anti-CD3 antibody.
(3) The trifunctional fusion protein of the present invention fuses with the FC protein to solve the problem of short half-life of the BiTE antibody.

### BRIEF DESCRIPTION OF THE DRAWINGS

Fig. 1 is the structural schematic diagram one of the trifunctional fusion protein of the present invention.
Fig. 2 is the structural schematic diagram two of the trifunctional fusion protein of the present invention.
Fig. 3 is the structural schematic diagram three of the trifunctional fusion protein of the present invention.
Fig. 4 is the structural schematic diagram four of the trifunctional fusion protein of the present invention.
Fig. 5 is a schematic diagram of the clone construction method.
Fig. 6 shows the results of a FACS assay for detecting binding of samples such as QP146134233424 to CHOS-CLDN18.2 cells.
Fig. 7 shows the results of a FACS assay for detecting binding of samples such as QP146134233424 to Jurkat cells.
Fig. 8 shows the results of an experiment in which samples such as QP146134233424 inhibiting the TGFβ-induced SMAD3 phosphorylation signaling pathway.
Fig. 9 shows the results of QP146134233424-mediated T cell killing of human gastric cancer NUGC4-CLDN18.2 cells.
Fig. 10 shows the results of QP146134233424-mediated PBMC cell killing of human gastric cancer NUGC4-CLDN18.2 cells.
Fig. 11 shows the inhibitory effect of QP146134233424 on tumor growth in the Mixeno animal pharmacodynamic model of HCC827-CLDN18.2

### EMBODIMENT FOR THE INVENTION

The technical solution of the present invention is further described below in conjunction with specific embodiments.

The experimental method without specific conditions in the present embodiment is generally in accordance with conventional conditions, or in accordance with the conditions recommended by the manufacturer of raw material or commodity. Examples are Molecular Cloning, Laboratory Manual, Cold Spring Harbor Laboratory, Current Protocols In Molecular Biology, Cell Biology and so on. Reagents without specific sources are conventional reagents purchased in the market.

The term "antibody" refers to an immunoglobulin molecule generally composed of two identical pairs of polypeptide chains, and each pair comprises one "light" (L) chain and one "heavy" (H) chain. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain CL.

The term "Fab fragment" is a fragment of antigen binding (Fab), which refers to an antibody fragment consisting of VL, VH, CL and CH1 domains.

Herein, Fc segment, Fc fragment and Fc have the same meaning. The term "Fc" refers to a fragment crystallizable, usually composed of a pair of CH2 and CH3 domains and devoid of antigen binding activity, that is the site for interaction of the antibody with effector molecules or cell surface Fc receptors. Since Fc may be composed of two chains, for convenience of description, the two chains of Fc fragment are described herein as A chain and B chain, and A chain and B chain may have the same structure or be mutated respectively, which is not particularly limited in the present invention.

Tumor-associated antigen (TAA) refers to the antigen molecules present on tumor cells or normal cells, including embryonic proteins, glycoprotein antigens, squamous cell antigens, etc., which are commonly used in clinical diagnosis of tumors. Tumor-associated antigens are not specific to tumor cells, but can be synthesized by normal cells in trace amounts, and are highly expressed when tumor cells proliferate, so they are called "associated antigens".

In the trifunctional fusion protein comprising a tumor-associated antigen (TAA) antibody, a TGF-β inhibitor, and a CD3 antibody of the present invention, the TAA antibody specifically targets the molecule to the tumor, and by introducing the TGF-β inhibitor into the antibody molecule, the TGF-β signal can be blocked and the infiltration and activation of T cells in the tumor microenvironment can be promoted, and the anti-CD3 antibody can exert a T cell killing effect.

The structure of the tumor-associated antigen (TAA) antibody, TGF-β inhibitor, or CD3 antibody is not particularly limited in the present disclosure.

The antibody or polypeptide fragment that binds to the tumor-associated antigen (TAA) can be a monovalent or multivalent molecule, and the bound tumor antigen or specific mutation thereof includes, but is not limited to, surface antigens such as CD20, CD19, CD38, CD30, CD52, slamf7, GD2, CD24, CD47, CD133, CD239, CD276, etc., and targets such as CEA, Epcam, Trop2, TAG72, MUC1, MUC16, mesothelin, folrl, CLDN18.2, PDL1, EGFR, EGFR VIII, C-MET, HER2, FGFR2, FGFR3, PSMA, PSCA, EphA2, ADAM17, 17-A1, NKG2D ligands, MCSP, LGRS, SSEA3, SLC34A2, BCMA, GPNMB, etc., and the antibody form includes Fab, ScFv, VHH, or peptide fragments.

The anti-CD3 antibody is monovalent and includes, but is not limited to, OKT3, SP34, UCTH1 and derivatives thereof or other specific antibodies, antibody fragments, single domain antibodies, nanobodies, and humanized versions thereof that bind to CD3.

The TGF-β inhibitor is a monovalent or multivalent polypeptide molecule capable of inhibiting the TGFβ pathway, which includes, but is not limited to, human TGFβRII extracellular domain and derivatives thereof that maintain binding to human TGFβ, human TGFβRI extracellular domain and derivatives thereof that maintain binding to human TGFβ, antibodies or polypeptide fragments that inhibit TGFβ or TGFβ receptors of the TGFβ pathway, or combinations thereof.

The trifunctional fusion protein of the present invention further comprises a linking sequence, which is an amino acid sequence with low immunogenicity, and the linking sequence links each component that composes the trifunctional fusion protein. In a specific embodiment, a suitable linking sequence such as (GS)n, (GSGGS)n, (GGGS)n, (GGGGS)n may be selected, and n is usually not greater than 5.

The components composing the trifunctional antibody can be selected in the form of chimeric, humanized, whole human, or single chain antibodies.

### I. Cloning and expression of fusion protein

### 1. Clone construction method:

1.1 Clones were designed as shown in Table 1, and full-length expression vectors were constructed. Designed proteins shown in Table 1 are the trifunctional fusion proteins of CLDN18.2, TGF-β RII and OKT3. CLDN18.2 (or claudin 18.2) was selected as the target of TAA antibody, human TGF β RII was selected as the TGF-β inhibitor, and OKT3 was selected as the target of CD3 antibody. Human IgG4 was selected as the FC fragment.

A total of 7 proteins were designed in Table 1, and the protein numbers were: QP32133212, QP34133414, QP34153416, QP34173418, QP34193420, QP34213422, and QP146134233424, respectively. For convenience of description, the seven proteins are abbreviated as protein-1, protein-2, protein-3, protein-4, protein-5, protein-6, and protein-7, respectively. The antibody Fc fragments were mutated by Knob-into-Hole (KiH) technology to obtain a heterodimer with correctly assembled heavy chains. The structures of protein-1 and protein-2 are similar, but the positions of the "hole" and "knob" structures formed by mutations on the two chains of the FC fragment are opposite. The same goes for Protein-3 and protein-4, protein-5 and protein-6.

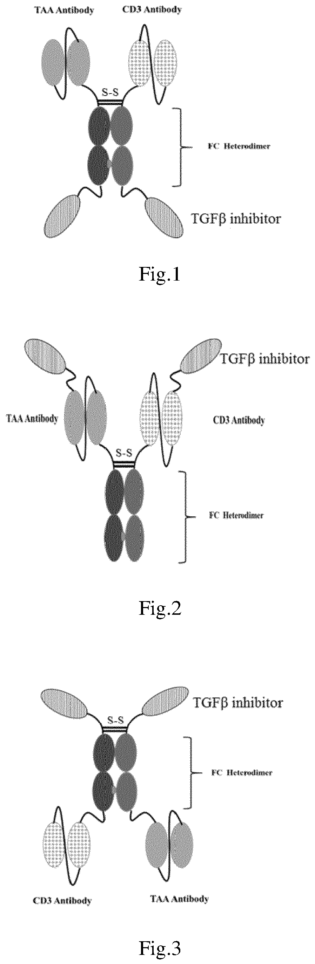

The structures of protein-1 and protein-2 can be seen in Fig. 1. In the fusion protein shown in Fig. 1, the C-termini of the two chains of the FC fragment are each fused with a TGF-β inhibitor, the N-terminus of one chain of the FC fragment is fused with the VH and VL domains of the anti-CLDN18.2 antibody, and the N-terminus of the other chain is fused with the VH and VL domains of the anti-OKT3 antibody, the antibody fragments fused to the N-termini of the FC fragment are all in the form of a single chain antibody (scFv), i.e., the VH and VL domains are linked by a linking sequence to form a single chain.

The structures of protein-3 and protein-4 can be seen in Fig. 2. In the fusion protein shown in Fig. 2, the N-terminus of one chain of the FC fragment is fused to the VH and VL domains of the anti-CLDN18.2 antibody, the N-terminus of the other chain is fused to the VH and VL domains of the anti-OKT3 antibody, and the VH and VL domains of the anti-CLDN18.2 antibody and the anti-OKT3 antibody are respectively connected by a linking sequence to form a single chain. Two TGF-β inhibitors are fused to the N-terminus of anti-CLDN18.2 antibody and anti-OKT3 antibody, respectively.

The structures of protein-5 and protein-6 can be seen in Fig. 3. In the fusion protein shown in Fig. 3, the N-termini of two chains of the FC fragment are each fused with a TGF-β inhibitor, the C-terminus of one chain of the FC fragment is fused to the VH and VL domains of the anti-OKT3 antibody, and the C-terminus of the other chain is fused to the VH and VL domains of the anti-CLDN18.2 antibody. The antibody fragments at the C-termini of the FC fragment are all in the form of single chain antibodies.

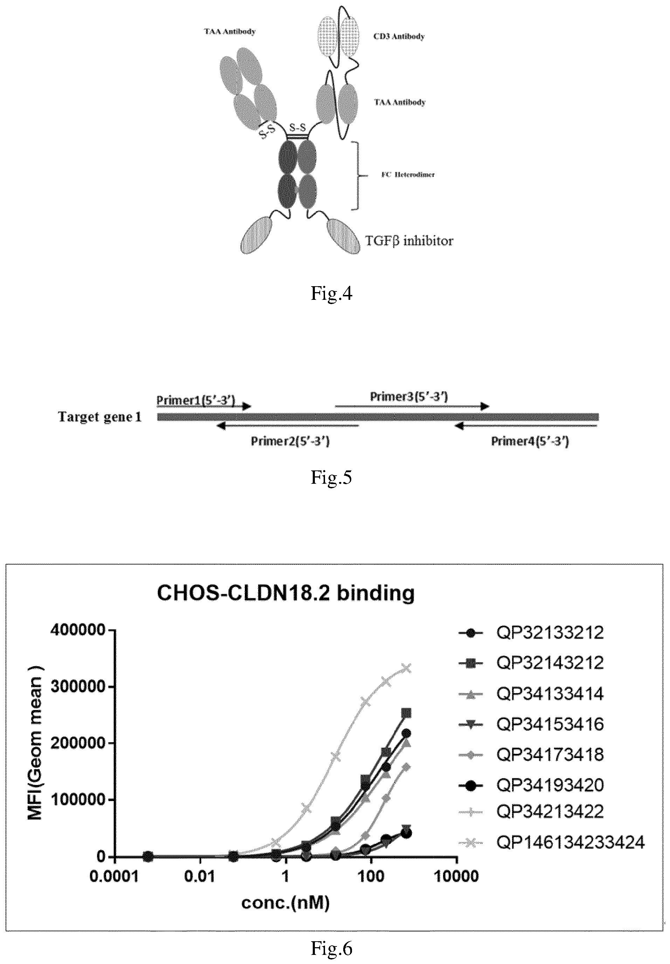

The structure of protein-7 can be seen in Fig. 4. In the cloning design of protein-7, three polypeptide chains were designed, and the protein formed the structure shown in Fig. 4. In the fusion protein shown in Fig. 4, the N-terminus of one chain of the FC fragment is fused with Fab fragment (comprising VH, VL, CH1 and CL domains) of anti-CLDN18.2 antibody; the N-terminus of the other chain of the FC fragment is fused with VH and VL domains of anti-CLDN18.2 antibody, and anti-OKT3 antibody (VH and VL domains) is fused to the N-terminus of anti-CLDN18.2 antibody. Both the anti-CLDN18.2 antibody and the anti-OKT3 antibody are scFvs. The C-termini of the FC are fused with a TGF-β inhibitor, respectively. The protein structure shown in Fig. 4 is fused with two TAA antibodies against the same target, and in some embodiments, two TAA antibodies against different targets can be selected.

The anti-CD3 antibody with a strong affinity to CD3 is likely to cause cytotoxicity. The anti-CD3 antibody with a moderate affinity to CD3 can be selected to adjust the effective dose of the anti-CD3 antibody to make it comparable to that of the TGF-βRII. The structure shown in Fig. 1 can be achieved by selecting anti-CD3 antibodies with moderate affinity to CD3. In Fig. 2, the anti-CD3 antibody is located inside the fusion protein, and in Fig. 3, the anti-CD3 antibody is located at the C-terminus of the FC fragment, both of which are beneficial to reduce the affinity with CD3.

**Table 1: Cloning designs for fusion proteins**

| **Clone construction** | | **Clone Number** | **Protein number** | **Protein Serial Numbe r** |
|---|---|---|---|---|
| SEQ ID NO: 1 | CLDN18.2 VH-VL-FC(hIgG4-hole)- TGFβRII | QD3213 | QP321332 12 | Protein-1 |
| SEQ ID NO:2 | OKT3 VH-VL-FC(hIgG4-knob)- TGFβRII | QD3212 | | |
| SEQ ID NO:3 | CLDN18.2 VH-VL-FC(hIgG4-knob)- TGFβRII | QD3413 | QP341334 14 | Protein-2 |
| SEQ ID NO:4 | OKT3 VH-VL-FC(hIgG4-hole)- TGFβRII | QD3414 | | |
| SEQ ID NO:5 | TGFβRII-CLDN18.2 VH-VL- FC(hIgG4-hole) | QD3415 | QP341534 16 | |
| SEQ ID NO:6 | TGFβRII-OKT3 VH-VL-FC(hIgG4- knob) | QD3416 | | Protein-3 |
| SEQ ID NO:7 | TGFβRII-CLDN18.2 VH-VL- FC(hIgG4-knob) | QD3417 | QP341734 18 | Protein-4 |
| SEQ ID NO:8 | TGFβRII-OKT3 VH-VL-FC(hIgG4- hole) | QD3418 | | |
| SEQ ID NO:9 | TGFβRII-FC(hIgG4-hole)-CLDN18.2 VL-VH | QD3419 | QP341934 20 | Protein-5 |
| SEQID NO:10 | TGFβRII-FC(hIgG4-knob)-OKT3 VL- VH | QD3420 | | |
| SEQID NO: 11 | TGFβRII-FC(hIgG4-knob)-CLDN18.2 VL-VH | QD3421 | QP342134 22 | Protein-6 |
| SEQID NO:12 | TGFβRII-FC(hIgG4-hole)-OKT3 VL- VH | QD3422 | | |
| SEQID NO:13 | CLDN18.2 VL-CL | QD 1461 | QP146134 233424 | Protein-7 |
| SEQID NO:14 | CLDN18.2 VH-CH1-FC(hIgG4-hole)- TGFβRII | QD3423 | | |
| SEQID NO:15 | OKT3 VH-VL-CLDN18.2 VH-VL- FC(hIgG4-knob)-TGFβRII | QD3424 | | |

The sequence listing of the present invention also relates to the following sequence information: SEQ ID NO:16 is TGFβRII (ECD), SEQ ID NO:17 is FC-Hole, SEQ ID NO:18 is FC-knob, SEQ ID NO: 19 is OKT3-VH, and SEQ ID NO:20 is OKT3-VL.

### 1.2 Primer design:

Using online software DNAWorks, v3.2.4, http://helixweb.nih.gov/dnaworks/, as shown in Fig. 5, multiple primers were designed for synthesis fragments containing recombinant desired gene.

### 1.3 Splicing of fragments:

According to the operating instructions of TaKaRa Primer STAR GXL DNA polymerase, PCR amplification was carried out to obtain the desired gene fragments by using the multiple primers designed above.

The first step PCR: 50 µL of PCR reaction system including 10 µL of PrimerSTAR GXL Buffer (5 ×); 4 µL dNTP Mixture (2.5 mmol . L⁻¹); 1 µL each primer as shown above; 1 µL PrimeSTAR GXL DNA Polymerase. PCR reaction conditions were 98°C for 2 min, 98°C for 20 s, 55°C for 15 s, 68°C for 30 s, 30 cycles; 68°C for 5 min.

The second step PCR: using the PCR product from the first step as a template, PCR amplification was performed using the first and last primers under the same conditions as the first step. PCR was used to construct and amplify the target fragments.

### 1.4 Construction and enzyme digestion of expression vector pQD:

Using the character of some special restriction enzymes, such as the BsmBI, that their recognition sequence and enzyme cleavage site are different, the expression vector with the signal peptide fragment was designed and constructed, and the expression vector was recorded as pQD. The vector was digested by BsmBI enzyme and the gum was cut for recovery.

### 1.5 Recombinant construction of expression vector:

The recombinant target gene fragment and the expression vector pQD with signal peptide fragment which was digested by BsmBI and recovered were added into DH5α competent cells according to the ratio of 3:1, respectively, then subjected to ice bath at 0°C for 30min, and heat shock at 42°C for 90s. 5 times the volume of LB medium was added, and incubated at 37°C for 45min, coated on LB-Amp plate, and cultured at 37°C overnight. Single clones were picked and sequenced to obtain each target clone.

### 2. Protein expression:

The culture density of 293E cells was maintained between (0.2-3) × 10⁶/ml, and the cells to be transfected were centrifuged and the medium was replaced one day before transfection, and the cell density was adjusted to (0.5-0.8) × 10⁶/ml. On the day of transfection, the cell density of 293E cells ranged from 1 to 1.5 × 10⁶/ml. Plasmid and transfection reagent PEI were prepared. The amount of plasmid to be transfected was 100 µg/100ml cells, and the mass ratio of PEI and plasmid was 2:1. The plasmid and PEI were mixed, and placed for 15 min, not more than 20 min. The mixture of plasmid and PEI was slowly added to the 293E cells and incubated in a shaker at 8% CO₂, 120rpm, 37°C. On the next day of transfection, 2mM VPA and 10× feed medium containing 2mM glucose + 0.2mM L-Glutamin were added. On the fifth to sixth day of transfection, the cell supernatant was collected by centrifugation at 4700rpm for 20min in a horizontal centrifuge for purification.

### II. Purification of fusion protein

### 1. Protein affinity chromatography:

After high-speed centrifugation of the cell culture solution, the supernatant was taken and subjected to affinity chromatography using a GE Protein A chromatography column. 1×PBS (pH7 .4) was used as the equilibrium buffer of chromatography. After cell supernatant loading and binding, the column was washed with PBS until the UV return to the baseline, then the target protein was eluted with elution buffer 0.1M glycine (pH3.0), and the pH was adjusted to neutral by Tris for preservation.

### 2. Protein ion exchange chromatography:

The pH of the product obtained by affinity chromatography was adjusted to 1~2 pH units below or above pI, and it was diluted appropriately to control the conductance of sample below 5 ms/cm. Using conditions including suitable corresponding pH buffer solutions such as phosphate buffer and acetic acid buffer, conventional ion exchange chromatography methods such as anion exchange or cation exchange were used for NaCl gradient elution under corresponding pH conditions. According to the SDS-PAGE, the collection tubes where the target proteins were present were selected for storing.

### 3. Protein size exclusion chromatography:

The product obtained by ion exchange was concentrated by ultrafiltration and then subjected to size exclusion chromatography, such as separation by GE Superdex200 gel, so as to remove possible polymers and other components and obtain the target product with high purity. The purity analysis of the obtained protein can be analyzed by SDS-PAGE and SEC-HPLC assays. Protein concentration was determined by UV spectrophotometry.

As shown in Table 2, the transient expression level of fusion protein in 293 cells is about 10-76mg/L.

**Table 2: Transient expression level of fusion protein (mg/L)**

| **Protein name** | **Protein yield (mg/L)** |
|---|---|
| QP32133212 | 9.90 |
| QP34133414 | 24.81 |
| QP34153416 | 76.30 |
| QP34173418 | 66.01 |
| QP34193420 | 39.49 |
| QP34213422 | 32.03 |
| QP146134233424 | 19.71 |

### III. FACS Detection of CLDN18.2 binding activity of samples

### Experimental steps:

CHOS-CLD18.2 cells were seeded in a U-shaped 96-well plate at 1E5/well, washed once with cold PBS, and centrifuged at 1200rpm for 3min. After washing, 3% FBS/PBS blocking solution was added at 200 µL/well, and incubated on ice for 1h. After blocking, the plate was centrifuged at 1200rpm for 3min, and the supernatant was removed. Samples with different concentrations were added and incubated on ice for 2h, the plate was washed with cold PBS for 3 times. PE-anti human FC antibody was added for incubation according to the dilution ratio of 1:200, 50 µl/well, after evenly mixed, the plate was incubated on ice for 1h, and washed with cold PBS for 3 times. The cells were resuspended in PBS at 200 µl/well, the mean fluorescence value was read by FACS instrument, and the results were analyzed using graphpad prism software. The results are shown in Fig. 6.

### IV. FACS Detection of CD3 Antibody Activity

### Experimental steps:

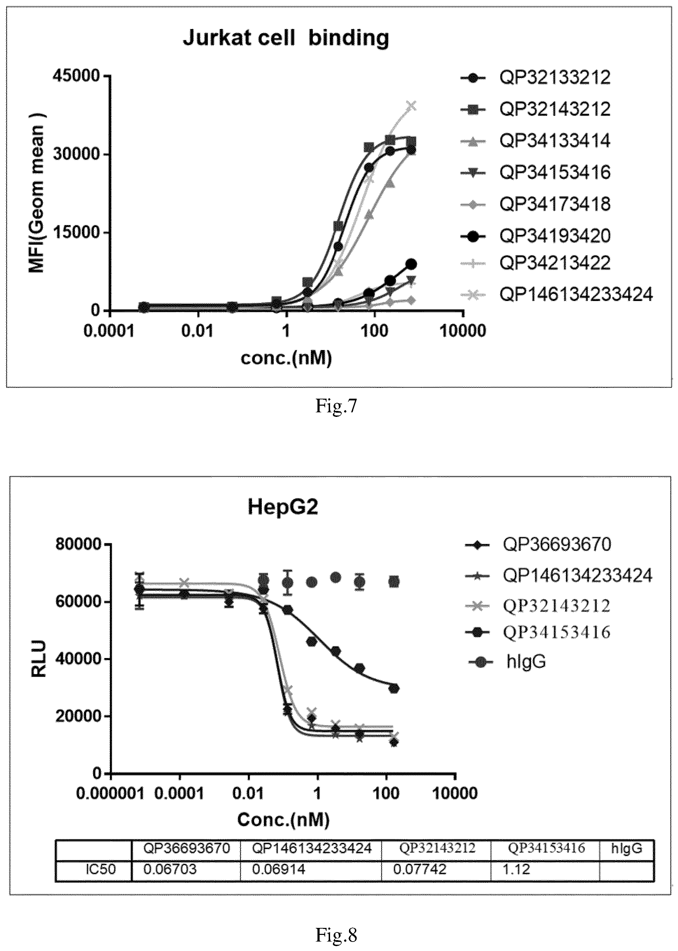

Jurkat cells were seeded in a U-shaped 96-well plate at 1E5/well, washed once with cold PBS, and centrifuged at 1200rpm for 3min. After washing, 3% FBS/PBS blocking solution was added at 200 µL/well, and incubated on ice for 1h. After blocking, the plate was centrifuged at 1200rpm for 3min, and the supernatant was removed. Samples with different concentrations were added and incubated on ice for 2h, the plate was washed with cold PBS for 3 times. PE-anti human FC antibody was added for incubation according to the dilution ratio of 1:200, 50 µl/well, after evenly mixed, the plate was incubated on ice for 1h, and washed with cold PBS for 3 times. The cells were resuspended in PBS at 200 µl/well, the mean fluorescence value was read by FACS instrument, and the results were analyzed using graphpad prism software. The results are shown in Fig. 7.

### V. SMAD3 reporter gene inhibition assay

Experimental objective: To determine that TGFBRII fragments can inhibit TGFβ-induced SMAD3 phosphorylation.

### Experimental steps:

In this experiment, TGFβRII function was determined by expression of the SMAD3-luciferase reporter gene in HepG2/4T1 cells.

HepG2 cells were cultured in MEM complete medium containing 10% FBS and passaged every 2-3 days. On the first day of the experiment, cells were seeded on a 96-well plate at a density of 25,000 cells per well, and cultured for 24h at 37°C and 5% CO₂. The medium in the cell culture plate was discarded the next day and 100ng 3TP-Lux plasmids were transfected per well. The cells were further cultured for 24h at 37°C, 5% CO₂. 6h before adding the sample to be tested, the complete medium in the 96-well plate was discarded, and 80 µl of incomplete medium (MEM + 0.5%FBS) was added to each well. After 6 hours, 10 µl of human TGF-β1 solution prepared in incomplete medium was added, with a final concentration of 2ng/ml and 10 µl of test sample, with a final concentration of 500, 50, 5, 0.5, 0.05, 0.005, 0.0005 and 0nM. Human TGF-β1 solvent was used as a control, and the cells were further cultured for 18h under the conditions of 37°C, 5% CO₂. Then 100 µl prepared fluorescein substrate ONE-GloTM Luciferase Assay system (promega, E6110) was added to each well, placed in dark at room temperature for 10 minutes, then the luminescence signal value was read in a multifunctional microplate reader. The IC50 values of the samples to be tested were calculated using data processing software Graphpad Prism 5.0. The results are shown in Fig. 8.

### VI. Detection of cytotoxicity of T cells killing tumor cells

From the above results, it can be seen that QP146134233424 (protein-7) has the best effect, so protein-7 was selected for cytotoxicity detection.

Experimental objective: To determine that the test molecule of the present invention can achieve the function of activating T cells to kill target cells by bridging TCR receptor on T cell by targeting CD3 at one end, and bridging target cell at the other end by targeting target cell surface antigen.

### Experimental method:

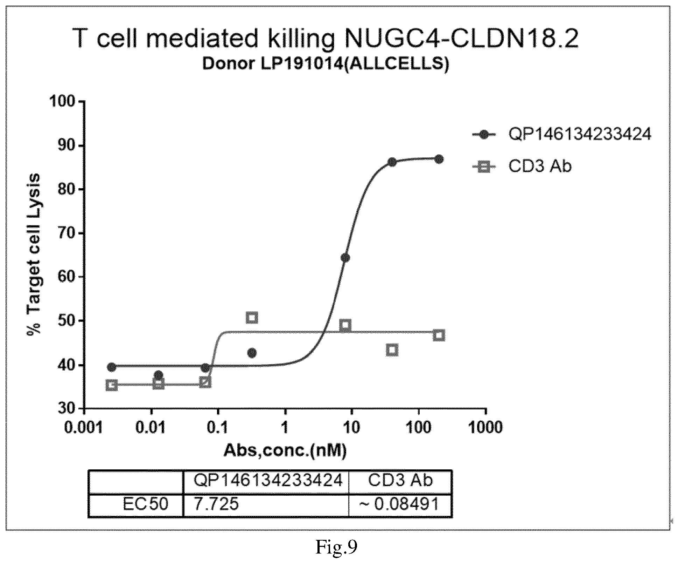

Human gastric cancer cell line NUGC4-CLDN18.2 with high expression of CLDN18.2 was selected as target cells. Effector cells were human CD4+ T cells sorted from human PBMCs (EasySep^{™} Human CD4+ T Cell Isolation Kit, Stemcell 17952). PBMCs were resuscitated with RPMI-1640 + 20% FBS, passed through 37µm cell screen, and the cells were resuspended in EasySep^{™} Buffer to 5 × 10⁷ cells/ml. CD4+ T Cell Isolation Cocktail was added at 50 µl/ml, incubated at room temperature for 5 min. Magnetic Particles were shook and mixed for 30 s, added into the tube at 50 µl/ml, mixed well, and incubated at room temperature for 5 min. EasySep^{™} Buffer was added to make the total volume of liquid in the tube 2.5 ml, gently blew up and down to mix for 2-3 times. The tube was put into the magnetic pole, and incubated at room temperature for 2.5min. The tube was turned upside down for 2~3s, and the suspension containing CD4+ T cells was poured into a new 15 ml centrifuge tube without shaking or sucking off the droplets remaining at the nozzle. The cell suspension was centrifuged at 300×g for 5 min, and the cells were collected as CD4+ T cells. According to the E:T ratio of 5:1, different concentrations of antibodies were added and cultured at 37°C, 5% CO₂ for 48h. CytoTox 96^{®} Non-Radioactive Cytotoxicity Assay (Promega, G1780 -1000 assays) was used to detect LDH in cell culture supernatant and quantify the percentage of cytotoxicity. The maximum lysis of target cells (100%) was all LDH that are released from cell lysis caused by 1% Triton X-100 treatment of target cells. Control wells were set up as target cell spontaneity, effector cell spontaneity, target cell + effector cell spontaneity, etc. Data were analyzed according to the formula: percent of killing =[(experimental well release - effector cell spontaneous release - target cell spontaneous release)/(maximum target cell lysis - target cell spontaneous release)] × 100. The results are shown in Fig. 9. It can be seen from Fig. 9 that QP146134233424 (protein-7) has a good function of activating T cells to kill target cells.

### VII. Detection of cytotoxicity of antibody-mediated PBMC killing tumor cells

### Experimental method:

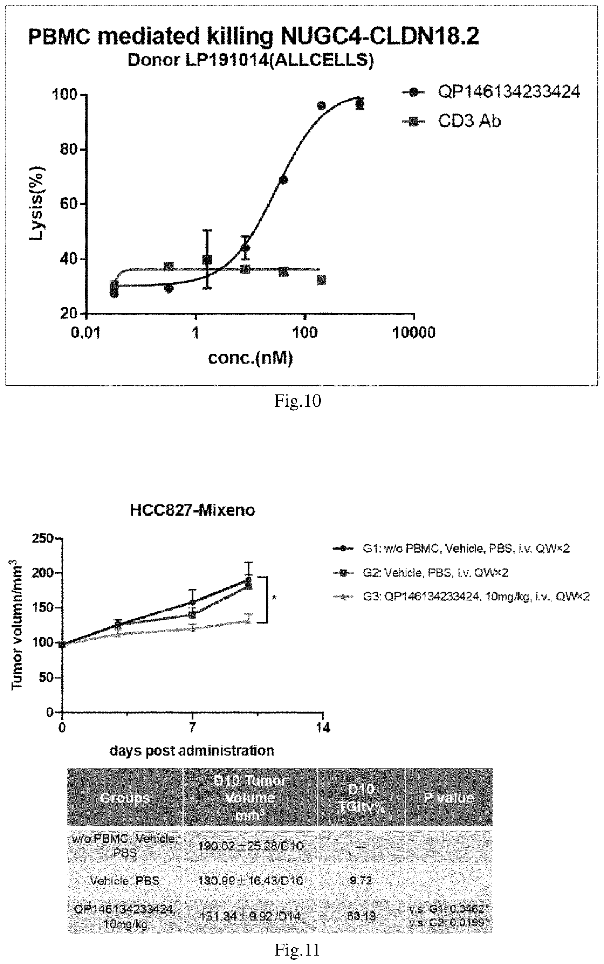

Human gastric cancer cell line NUGC4-CLDN18.2 with high expression of CLDN18.2 was selected as target cells. Effector cells were human PBMCs. According to the E:T ratio of 10:1, different concentrations of antibodies were added and cultured at 37°C, 5% CO₂ for 48h. CytoTox 96^{®} Non-Radioactive Cytotoxicity Assay (Promega, G1780 -1000 assays) was used to detect LDH in cell culture supernatant and quantify the percentage of cytotoxicity. The maximum lysis of target cells (100%) was all LDH that are released from cell lysis caused by 1% Triton X-100 treatment of target cells. Control wells were set up as target cell spontaneity, effector cell spontaneity, target cell + effector cell spontaneity, etc. Data were analyzed according to the formula: percent of killing =[(experimental well release - effector cell spontaneous release - target cell spontaneous release)/(maximum target cell lysis - target cell spontaneous release)] × 100. The results are shown in Fig. 10. The results show that using QP146134233424 (protein-7) can obtain good killing tumor cells function.

### VIII. Efficacy on Animal

The inhibitory effect of anti-claudin18.2-TGFβRII-CD3 fusion protein on tumor growth was evaluated in an immune reconstituted mouse model.

Since CD3 antibody does not recognize mouse CD3, an immune reconstituted mouse model (PBMC-graft NCG) was used to evaluate the growth inhibitory effect of claudin18.2-TGFβRII-CD3 fusion protein on claudin18.2 expressing tumor cells. HCC827-claudin18.2 cells were cultured in RPMI1640 medium containing 10% fetal bovine serum + 1× sodium pyruvate + 2mM glutamax + 1 mg/ml G-418. HCC827 cells in exponential growth phase were harvested and resuspended in PBS to the appropriate concentration for inoculation. Each experimental mouse was subcutaneously inoculated with 5 × 10⁶ of HCC827 cells on the right back, and the growth of the tumor was regularly observed. When the tumor grew to an average volume of about 100mm³, the mice were randomly divided into groups according to the tumor size and the body weight of the mice for administration. The day of tumor cell inoculation was defined as day 0. Group G1 was a blank control, which was injected with PBS buffer. On the day of grouping, each mouse in group G2 and G3 was intraperitoneally injected with PBMCs in an amount of 5 × 10⁶ cells/mouse. After injection of PBMCs, administration was started. The dosing regimen was as follows:

| Group | Number of animals | Treatment group | Dose (mg/kg) | Mode of administration | Cycle of administration |
|---|---|---|---|---|---|
| 1 | 9 | w/o PBMC+vehicle | -- | i.p. | Q7D × 3 |
| 2 | 9 | PBMC+vehicle | -- | i.p. | Q7D × 3 |
| 3 | 9 | PBMC+QP146134233424 | 10 | i.p. | Q7D × 3 |

The dosing volume was 10 µl/g body weight.

The body weight and tumor volume of the mice were observed twice a week, the long and short diameters of the tumor was measured with a vernier caliper, and the tumor growth was calculated and recorded according to the formula: tumor volume (mm³) = 0.5 × (a×b²), and the tumor growth curve was drawn. The results are shown in Fig. 11. The results show that using QP146134233424 (protein-7) can obtain better tumor inhibition effect.

From the above results, it can be seen that in the present invention, the trifunctional fusion protein design that fuses tumor related antigen (TAA) antibody, TGF-β inhibitor, and CD3 antibody is feasible, and QP146134233424 has been verified to have a better tumor inhibition effect in the present invention. The trifunctional fusion of the present invention is not limited to the forms listed in the above examples, and any protein that trifunctionally fuses tumor-associated antigen (TAA) antibody, TGF-β inhibitor and CD3 antibody should be included in the protection scope of the present invention.

## Claims

1. A trifunctional fusion protein comprising a tumor-associated antigen (TAA) antibody, a TGF-β inhibitor and a CD3 antibody.

2. The trifunctional fusion protein comprising a tumor-associated antigen (TAA) antibody, a TGF-β inhibitor and a CD3 antibody according to claim 1, wherein the fusion protein has the following general structural formula:
{[TAA antibody]n, [CD3 antibody]x, [TGF-β inhibitory molecule]m}, wherein, n〉=1, x = 1, m〉=1;
wherein,
the [TAA]n refers to an antibody or polypeptide fragment that binds to a tumor-associated antigen in a monovalent or multivalent manner;
the [CD3]x refers to an antibody or polypeptide fragment that binds to CD3 in a monovalent manner;
the [TGF-β inhibitory molecule]m refers to an antibody or polypeptide fragment that binds to TGF-β in a monovalent or multivalent manner.

3. The trifunctional fusion protein comprising a tumor-associated antigen (TAA) antibody, a TGF-β inhibitor and a CD3 antibody according to claim 1, wherein the target of the TAA antibody includes: CD20, CD19, CD38, CD30, CD52, slamf7, GD2, CD24, CD47, CD133, CD239, CD276, CEA, Epcam, Trop2, TAG72, MUC1, MUC16, mesothelin, folrl, CLDN18.2, PDL1, EGFR VIII, C-MET, HER2, FGFR2, FGFR3, PSMA, PSCA, EphA2, ADAM17, 17-A1, NKG2D ligands, MCSP, LGRS, SSEA3, SLC34A2, BCMA, or GPNMB;
the target of the CD3 antibody includes: OKT3, SP34 or UCTH1;
the TGF-β inhibitor includes: human TGF-βRII extracellular region and a derivative thereof or human TGF-βRI extracellular region and a derivative thereof.

4. The trifunctional fusion protein comprising a tumor-associated antigen (TAA) antibody, a TGF-β inhibitor and a CD3 antibody according to claim 1, wherein the fusion protein further comprises an FC segment; the FC segment is selected from the group consisting of Human IgG1 FC, Human IgG2 FC, Human IgG3 FC, Human IgG4 FC and a variant thereof; and the FC segment comprises an A chain and a B chain, and the variant includes the following forms:
| Combination Number | FC | Heterodimer mutation (Eu numbering) |
|---|---|---|
| 1 | FC-A chain | T366Y |
| | FC-B chain | Y407T |
| 2 | FC-A chain | T366W |
| | FC-B chain | T366S/L368A/Y407V |
| 3 | FC-A chain | S354C/T366W |
| | FC-B chain | Y349C/T366S/L368A/Y407V |
| 4 | FC-A chain | S364H/F405A |
| | FC-B chain | Y349T/T394F |
| 5 | FC-A chain | T350V/L351Y/F405A/Y407V |
| | FC-B chain | T350V/T366L/K392L/T394W |
| 6 | FC-A chain | K392D/K409D |
| | FC-B chain | E356K/D399K |
| 7 | FC-A chain | ID221E/P228E/L368E |
| | FC-B chain | D221R/P228R/K409R |
| 8 | FC-A chain | K360E/K409W |
| | FC-B chain | Q347R/D399V/F405T |
| 9 | FC-A chain | K360E/K409W/Y349C |
| | FC-B chain | Q347R/D399V/F405T/S354C |
| 10 | FC-A chain | K370E/K409W |
| | FC-B chain | E357N/D399V/F405T |
| 11 | FC-A chain | F405L |
| | FC-B chain | K409R |
| 12 | FC-A chain | K360D/D399M/Y407A |
| | FC-B chain | E345R/Q347R/T366V/K409V |
| 13 | FC-A chain | Y349S/K370Y/T366M/K409V |
| | FC-B chain | E356G/E357D/S364Q/Y407A |
| 14 | FC-A chain | L351D/L368E |
| | FC-B chain | L351K/T366K |
| 15 | FC-A chain | GQPFRPEVHLLPPSREEMTKNQVSLTCLARGFYPKDIAV EWESNGQPENNYKTTPSRQEPSQGTTTFAVTSKLTVDK SRWQQGNVFSCSVMHEALHNHYTQKTISL |
| | FC-B chain | GQPREPQVYTLPPPSEELALNELVTLTCLVKGFYPSDIA VEWLQGSQELPREKYLTWAPVLDSDGSFFLYSILRVAA EDWKKGDTFSCSVMHEALHNHYTQKSLDR |
| 16 | FC-A chain | L368D/K370S |
| | FC-B chain | E357Q/S364K |
| 17 | FC-A chain | S354C/T366W/K409A |
| | FC-B chain | Y349C/T366S/L368A/Y407V/F405K |
| 18 | FC-A chain | S354C/T366W/F405K/K360EQ347E |
| | FC-B chain | Y349C/T366S/L368A/Y407V/Q347R/T394W |
| 19 | FC-A chain | T366W/K409A |
| | FC-B chain | T366S/L368G/Y407A/F405K |
| 20 | FC-A chain | knobs(T366W/F405K) |
| | FC-B chain | holes(T366S/L368G/Y407A/K409A) |
| 21 | FC-A chain | Q347A/S364K/T366V/K370T/K392Y/F405S/Y407V/K409W/ T411N |
| | FC-B chain | Q347E/Y349A/L351F/S364T/T366V//K370T/T394D/V397L/ D399E/D401Q/F405A/Y407S/K409R/T411R |
| 22 | FC-A chain | K274Q/N276K/Y300F/A339T/Q347A/S364K/T366V/K370T/ N384S/K392Y/V397M/F405S/Y407V/K409W/T411N/V422I/ H435R/Y436F |
| | FC-B chain | Q347E/Y349A/L351F/S364T/T366V//K370T/T394D/V397L/ D399E/D401Q/F405A/Y407S/K409R/T411R |

5. The trifunctional fusion protein comprising a tumor-associated antigen (TAA) antibody, a TGF-β inhibitor and a CD3 antibody according to claim 1, wherein the trifunctional fusion protein comprises: an FC segment, a TAA antibody, two TGF-β inhibitors, and a CD3 antibody;
the TAA antibody comprises: a first TAA antibody and a second TAA antibody, and the first TAA antibody comprises a Fab domain,
the second TAA antibody and the CD3 antibody are scFvs;
the FC segment comprises an A chain and a B chain; wherein the N-terminus of the A chain is linked to the first TAA antibody, and the C-terminus of the A chain is linked to one TGF-β inhibitor; the N-terminus of the B chain is linked to the second TAA antibody, and the C-terminus of the B chain is linked to the other TGF-β inhibitor; and the CD3 antibody is linked to the second TAA antibody.

6. The trifunctional fusion protein comprising a tumor-associated antigen (TAA) antibody, a TGF-β inhibitor and a CD3 antibody according to claim 1, wherein the target of the TAA antibody is CLDN18.2, the target of the CD3 antibody is OKT3; and the TGF-β inhibitor is human TGF-βRII extracellular domain.

7. The trifunctional fusion protein comprising a tumor-associated antigen (TAA) antibody, a TGF-β inhibitor and a CD3 antibody according to claim 6, wherein the fusion protein is obtained by the fusion of SEQ ID NOs: 13, 14 and 15.

8. The trifunctional fusion protein comprising a tumor-associated antigen (TAA) antibody, a TGF-β inhibitor, and a CD3 antibody according to claim 1, wherein the fusion protein further comprises a linking sequence which comprises one or more G4S.

9. Use of the trifunctional fusion protein comprising a tumor associated antigen (TAA) antibody, a TGF-β inhibitor, a CD3 antibody according to any one of claims 1 to 8 in the preparation of a medicament for inhibiting or treating cancer.

10. The use according to claim 9, wherein the cancer includes: myeloma, lymphoma, leukemia, small cell lung cancer, non-small cell lung cancer, melanoma, basal cell skin cancer, squamous cell skin cancer, dermatofibrosarcoma protuberans, Merkel cell carcinoma, glioblastoma, glioma, sarcoma, mesothelioma, or myelodysplastic syndrome, or a cancer or tumor derived from: colorectal, breast, ovary, pancreas, stomach, prostate, kidney, cervix, thyroid, endometrium, uterus, bladder, neuroendocrine, head and neck, liver, nasopharynx or testis.
